(19) Europäisches Patentamt
European Patent Office
Office européen des brevets

(11) **EP 1 357 143 A1**

(12) **EUROPEAN PATENT APPLICATION**

(43) Date of publication:
**29.10.2003 Bulletin 2003/44**

(21) Application number: **03252203.9**

(22) Date of filing: **08.04.2003**

(51) Int Cl.[7]: **C08G 77/14**, C08G 77/26, C08G 77/38, C08G 77/46, A61K 7/13, A61K 7/135

(84) Designated Contracting States:
**AT BE BG CH CY CZ DE DK EE ES FI FR GB GR HU IE IT LI LU MC NL PT RO SE SI SK TR**
Designated Extension States:
**AL LT LV MK**

(30) Priority: **22.04.2002 GB 0209134**

(71) Applicant: **THE PROCTER & GAMBLE COMPANY**
**Cincinnati, Ohio 45202 (US)**

(72) Inventors:
- **Glenn, Robert Wayne**
  **Virginia Water, Surrey, GU25 4HZ (GB)**
- **Godfrey, Simon Paul**
  **Uxbridge, Middlesex, UB8 1AR (GB)**
- **McMeekin, Anthony**
  **Bagshot, Surrey, GU19 5QY (GB)**
- **Stonehouse, Jonathan Richard**
  **Windlesham, Surrey, GU20 6HR (GB)**

(74) Representative: **Kohol, Sonia et al**
**Patent Department,**
**Procter & Gamble Technical Centers Limited,**
**Rusham Park,**
**Whitehall Lane**
**Egham, Surrey TW20 9NW (GB)**

(54) **Silicones**

(57) In a first aspect, the present invention relates organomodified silicones having substituents comprising oxygen, such that the oxygen content of the substituents is from 1% to 10% of the weight of the functionalized silicone and the content of the silicone backbone is from 67 to 95% by weight. In a further aspect, the invention relates to topical compositions comprising these organomodified silicones which deposit on a variety of fibers, especially hair, of differing levels of damage. In a further aspect, the invention relates to a hair treatment kit comprising the topical compositions.

EP 1 357 143 A1

## Description

### FIELD OF THE INVENTION

In a first aspect, the present invention relates functionalized silicones having defined structures. In a further aspect, the invention relates to topical compositions comprising these functionalized silicones; these compositions exhibit superior conditioning efficacy on both polar and non-polar substrates than previously known silicone based conditioners, especially where the substrate is hair that has been previously damaged through chemical treatments, such as occurs during permanent dyeing, bleaching and permanent waving. In a further aspect, the invention relates to a hair treatment kit comprising topical compositions according to the invention.

### BACKGROUND OF THE INVENTION

Oxidative dyeing, otherwise known as permanent coloring leads to irreversible physic-o-chemical changes to the hair. Typically, during this process, two components are mixed together prior to application to the hair. These components usually comprise an oxidising agent, such as hydrogen peroxide, and a dyeing material, such as oxidative dye precursors and couplers (buffered at a high pH, typically around 10). After contacting with the hair, the mixture is left for a period of time suitable to allow the required color transformation to occur, after which the hair becomes more hydrophilic versus non-colored hair due to irreversible chemical changes. While not wishing to be bound by theory, this change in hair hydrophilicity appears to be due, among other things, to the oxidation of the keratin-keratin cystine amino acids within the hair creating more hydrophilic cysteic acid amino acid residues and the removal by hydrolysis of nature's hydrophobic F-Layer. This coloring process is usually repeated regularly by consumers in order to maintain their desired hair color and color intensity and also to ensure that new hair growth has the same color as the older hair. As a consequence the hair changes polarity from a relatively hydrophobic surface near the scalp where it could be experiencing its first color, to a progressively more polar substrate at the hair tips, which may have been subjected to multiple coloring treatments. A discussion of oxidation dyeing of hair can be found in "The Science of Hair Care" by Charles Zviak, Marcel Dekker, New York, 1986.

These irreversible physicochemical changes can also manifest themselves as increased roughness, brittleness and dryness leading to less manageable hair. The use of conditioners within the coloring process is known. Conditioning materials can be added to the colorant product, or alternatively these can be supplied within the colorant kit as a separate conditioner, and can thereby be applied to the hair either during the coloring event or after the colorant has been rinsed. As described in EP 0 275 707, it is known to use aminosilicone for this purpose. However, it has also been established that, in the case of more polar hair, such as that obtained after successive oxidative colorings, aminosilicone deposition is greatly reduced and cannot provide the same level of benefit in hair condition as for non-oxidatively colored hair, especially when delivered within the harsh coloring environment. Without wishing to be bound by theory, the reason for this may be that there exists a surface energy incompatibility between the polar chemically damaged hair with the relatively non-polar aminosilicone leading to poorer adhesion.

Improving deposition evenness between more and less damaged hair represents a major improvement area to enable sufficient deposition at the more polar/damaged tips for a noticeable conditioning benefit where it is needed most without over-depositing at the roots. Moreover, this technical challenge represents an even bigger issue across differing consumer populations. For instance, the varying damage levels found in different individuals can result in the same silicone generating acceptable deposition in some cases, but over-deposition, with the accompanying negative sensory implications, in others (e.g. first time colorers vs. frequent colorers, brunettes vs. bleached blondes etc). Hence, a silicone active that deposits evenly across all hair types and damage levels is highly desirable.

Improving hair feel immediately after coloring is not the only desirable property of a colorant conditioner. After the coloring process human hair becomes soiled due to its contact with the surrounding environment and from the sebum secreted from the scalp. This soiling of the hair causes it to have a dirty feel and unattractive appearance and necessitates shampooing with frequent regularity. Shampooing cleans the hair by removing excess soil and sebum, but can leave the hair in a wet, tangled, and generally unmanageable state. Once the hair dries, it is often left in a dry, rough, lustreless, or frizzy condition due to the removal of the hair's natural oils and other natural or deposited conditioning and moisturizing components. Hair can also be left with increased levels of static upon drying which can interfere with combing and result in a condition commonly referred to as "fly-away-hair". These conditions tend to be exaggerated on hair which has been previously oxidatively colored.

A variety of approaches have been developed to alleviate these post-shampoo problems. These approaches range from post-shampoo application of hair conditioners such as leave-on or rinse-off products, to hair conditioning shampoos which attempt to both cleanse and condition the hair from a single product. Hair conditioners are typically applied in a separate step following shampooing. The hair conditioners are either rinsed-off or left-on, depending upon the type of product used. Polydimethylsiloxanes (PDMS) are often employed as conditioning materials in both shampoo

and conditioner applications to improve hair feel. However, it is known that, in the case of more hydrophilic hair obtained after oxidative coloring, PDMS deposition is greatly reduced, and cannot provide the same benefit in hair condition as for non-oxidatively colored hair.

Creating a conditioner that does not need to be applied every time the hair is washed would be highly advantageous and is not something that prior art compositions, including compositions defined in the above-mentioned art, currently allow. This is especially the case for the hydrophilic oxidatively damaged hair, typical of hair that has been permanently colored, which is much more vulnerable to subsequently further damage during the routine shampooing process. Having a durably deposited conditioner would enable round-the-clock protection to the hair. It is therefore highly desired that a conditioning active deposited during the coloring process remains on the hair during washing cycles in the days and weeks following coloring to provide a durable conditioning benefit.

Lastly, to obtain an improved conditioning effect, it is also important to ensure that enough silicone fluid deposits on each filament to meet consumer needs, i.e. that the absolute deposition of silicone fluid is sufficient for this purpose, both initially and long-term after subsequent shampooings.

Summarising some of the consumer needs discussed above, a dilemma faced by the present inventors was to produce a conditioner that deposits evenly and durably on hair of differing damage states, from undamaged, virgin hair, at the one extreme, to hair exposed to multiple oxidative dye treatments, at the other.

Attempts appear to have been made in the prior art to solve some of the problems discussed above. To be more specific, there has been a move away from using the highly hydrophobic PDMS-based silicones and towards using functionalized silicones, comprising functional groups such as amines, discussed above, and (among others) quaternary ammonium moieties. US 6,136,304, for example, discusses problems associated with conditioning compounds which are too easily rinsed from the hair. It also discusses application of conditioners to both virgin and damaged hair. The ethoxylated quaternary ammonium functionalized silicones proposed to achieve these aims, such as ABIL-QUAT 3272 (see Table 1, below) and ABIL-QUAT 3270 (both having the CTFA designation of quaternium-80), produced by the Goldschmidt Chemical Corporation, Hopewell, Va, are so hydrophilic, however, that they are rapidly washed off during subsequent shampooings. In other words, they do not achieve sufficient durability to meet consumer needs. In terms of polarity, these silicones are at the other end of the spectrum from the PDMS-type materials, but they are similarly non-durable and therefore unsuitable.

With the above discussion in mind, the invention will ideally provide functionalized silicones which are capable of depositing evenly on all types of hair which occur in today's human population, from undamaged, virgin hair, at the one extreme, to hair exposed to multiple oxidative dye treatments, at the other.

Additionally, the invention will ideally provide a functionalized silicone which deposits evenly over the whole length of a hair strand, including both lengths of uncolored scalp hair and hair previously colored with an oxidative colorant.

Additionally, the present invention will ideally provide durable silicone-conditioning agent for use on oxidation dyed hair, which does not wash off so rapidly that the conditioning benefit is lost to the consumer.

Additionally, the invention will ideally provide fiber treatment compositions comprising functionalized silicones according to the invention.

These and other features, aspects, and advantages of the present invention will become evident to those skilled in the art from a reading of the present disclosure.

## SUMMARY OF THE INVENTION

According to a first aspect of the invention, functionalized silicones of the pendant or graft type are provided having a viscosity in the range 400 to 150,000 mPa.s and incorporating one or more polar substituents selected from electron withdrawing, electron neutral, or electron donating groups with Hammett sigma para values between -1.0 and +1.5, wherein the one or more polar substituents comprise oxygen, such that the oxygen content of the summation of the one or more polar substituents is from 1% to 10% of the weight of the functionalized silicone and the silicone content is from 67 to 95% of the weight of the functionalized silicone.

As used herein, the term "fiber" includes strands of natural or synthetic materials. Non-limiting examples of natural materials are amino acid based materials, including protinaceous materials such as wool, human hair, including velus hair, and animal fur; cotton; cellulose and silk. Non-limiting examples of synthetic materials are polyester, nylon and rayon.

As used herein, the term "functionalized" silicone includes polydimethylsiloxanes (PDMS) in which at least one methyl group has been replaced by another group, which is preferably not hydrogen. The term "functional silicone" is synonymous with the term "functionalized silicone".

As used herein, the term "even" when used in relation to functionalized silicone deposition refers to the relative deposition on damaged as opposed to undamaged hair and means that the Deposition Evenness Value, as measured using the protocol hereinbelow, is at least 50%, preferably at least 60% and more preferably at least 70%. Phrases

such as "deposits evenly" and "even deposition" are to be interpreted accordingly.

As used herein, the term "durable" used in relation to functionalized silicone deposition means that the Durability Index, as measured by the Silicone Durablity Index Method protocol, hereinbelow, is at least 0.20, preferably greater than 0.50, more preferably greater than 0.75, and most preferably greater than 1.0. Phrases such as "deposits durably" and "durable deposition" are to be interpreted accordingly.

Hammett sigma para values are discussed in Römpp Chemie Lexikon, Georg Thieme Verlag, Stuttgart, New York, $9^{th}$ Edition, 1995 under "Hammett Gleichung".

The functionalized silicone structures claimed are such that they are capable of depositing evenly and durably on a fibrous substrate. Without wishing to be bound by theory, this is considered to be due to the above defined percentages of oxygen and silicone which impart the polarity characteristics needed to achieve both sufficient durability and deposition evenness simultaneously.

The term HLB value is known to the skilled person working in this technical area - see for esample Römpp Chemie Lexikon, Thieme Verlag, Stuttgart, $9^{th}$ Edition, 1995 under "HLB-Wert".

## DETAILED DESCRIPTION

All cited references are incorporated herein by reference in their entireties.

All percentages given herein are by weight of total composition unless specifically stated otherwise. All ratios given herein are weight ratios unless specifically stated otherwise.

All molecular weights given herein are weight average molecular weights, unless stated otherwise.

Except where specific examples of actual measured values are presented, numerical values referred to herein should be considered to be qualified by the word "about".

According to the invention, the one or more polar substituents comprise oxygen, such that the oxygen content (% oxygen) of the summation of the one or more polar substituents (not including the oxygen in the PDMS backbone) is from 1% to 10%, preferably from 2% to 9%, more preferably from 3% to 8% of the weight of the functionalized silicone and the silicone content is from 67 to 95%, preferably from 70% to 90%, and more preferably from 75, to 85% of the weight of the functionalized silicone. The silicone content or calculated percent silicone (% silicone) is defined as the average molecular weight of the PDMS backbone (consisting of silicon, oxygen and any directly attached methyl groups) divided by the average molecular weight of the whole polymer. Similarly, the overall oxygen content (% oxygen) is defined as the molecular weight of each oxygen atom multiplied by the average number of oxygen atoms present on the functionalized silicone (not including the oxygen in the PDMS backbone) and then divided by the average molecular weight of the whole polymer.

The present inventors have also established that the silicone fluid viscosity has an influence on the absolute deposition level, the level of durability and also the tactile sensorial feel of the deposited silicones. Advantageously, according to an embodiment of the invention, the silicone has a viscosity in the range 50 - 150,000 mPa.s. More advantageously, the viscosity is in the range 400- 100,000 mPa.s. More advantageously still, the viscosity is in the range 4000 - 25,000 mPa.s.

Below 50 mPa.s, the durability of the functionalized silicone is too low to be acceptable.

The deposition and durability of a functionalized silicone increase with increasing viscosity up to a plateau viscosity which was found to be above 400 mPa.s. While not wishing to be bound by theory, at this plateau viscosity the silicone is believed to provide sufficient resistance to contraction, "roll-up" and subsequent removal during the time frame of the rinsing process to improve deposition.

We have also established that above 4000 mPa.s the tactile feel of the deposited functional silicone is improved. Without being limited by theory, we believe this is due to the formation of a smoother morphology of the deposited structures versus the fluids between 400 and 4000 mPa.s.

Advantageously, the functionalized silicone according to the invention is an organomodified silicone of the pendant or graft type wherein the polar functional substituents are incorporated within or onto monovalent organic groups, $A^1$, $A^2$, $A^3$ and $A^4$ used hereinafter, as follows:

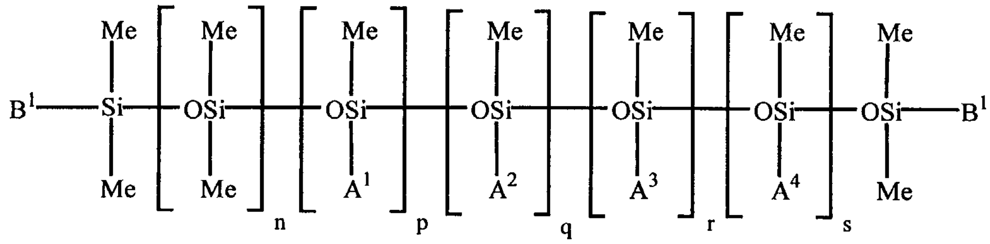

Also included are the organomodified silicones of the block copolymer type wherein these polar functional substituents are incorporated within or onto bivalent organic groups, $A^1$, $A^2$, $A^3$ and $A^4$ used hereinafter:

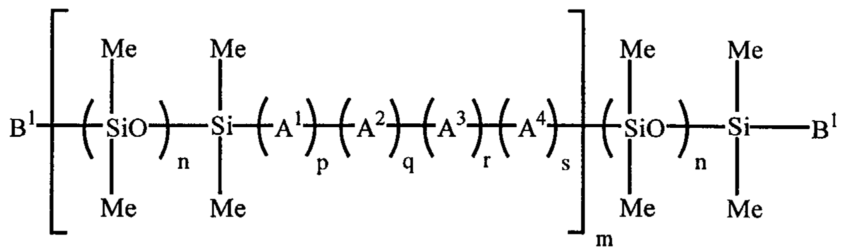

where Me is a methyl group, m is greater than or equal to 1, n is about 50 to 2000, p is about 0 to 50, q is about 0 to 50, r is about 0 to 50, s is about 0 to 50, wherein p + q + r + s is greater than or equal to 1, $B^1$ is H, OH, an alkyl or an alkoxy group and organic groups $A^1$, $A^2$, $A^3$ and $A^4$ are straight, branched or mono- or polycyclic aliphatic, mono or polyunsaturated alkyl, aryl, heteroalkyl, heteroaliphatic or heteroolefinic moieties comprising 3 to 150 carbon atoms together with 0-50 heteroatoms.

More advantageously, the polar substituents may be non-ionic, zwitterionic, cationic or anionic comprising, for example, groups $\alpha^1$, $\alpha^2$, $\alpha^3$, and $\alpha^4$ as defined below; S-linked groups including $S\alpha^1$, SCN, $SO_2\alpha^1$, $SO_3\alpha^1$, $SS\alpha1^1$, $SO\alpha^1$, $SO_2N\alpha^1\alpha^2$, $SN\alpha^1\alpha^2$, $S(N\alpha^1)$ $\alpha^2$, $S(O)(N\alpha^1)$ $\alpha^2$, $S\alpha^1(N\alpha^2)$, $SON\alpha^1\alpha^2$; O-linked groups including $O\alpha^1$, $OO\alpha^1$, OCN, $ON\alpha^1\alpha^2$; N-linked groups including $N\alpha^1\alpha^2$, $N\alpha^1\alpha^2\alpha^3$+, NC, $N\alpha^1O\alpha^2$, $N\alpha^1S\alpha^2$, NCO, NCS, $NO_2$, $N{=}N\alpha^1$, $N{=}NO\alpha^1$, $N\alpha^1CN$, $N{=}C{=}N\alpha^1$, $N\alpha^1N\alpha^2\alpha^3$, $N\alpha^1N\alpha^2N\alpha^3\alpha^4$, $N\alpha^1N{=}N\alpha^2$; other miscellaneous groups including COX, $CON_3$, $CON\alpha^1\alpha^2$, $CON\alpha^1CO\alpha^2$, $C({=}N\alpha^1)N\alpha^1\alpha^2$, CHO, CHS, CN, NC, and X, where:

$\alpha^1$, $\alpha^2$, $\alpha^3$, and $\alpha^4$ may be straight, branched or mono- or polycyclic aliphatic, mono or polyunsaturated alkyl, aryl, heteroalkyl, heteroaliphatic or heteroolefinic moiety comprising 3 to 150 carbon atoms together with 0-50 heteroatoms, especially 0, N, S, P.

X is F, Cl, Br, or I.

H is hydrogen, O is oxygen, N is nitrogen, C is carbon, S is sulfur, Cl is chlorine, Br is bromine, I is iodine, F is fluorine.

The above organomodified silicones of the pendant or block copolymer type may, according to the invention, also incorporate silicone branching groups including $MeSiO_{3/2}$, known as silsesquioxane or T groups, and $SiO_{4/2}$, known as Q groups by those skilled in the art.

Preferred polar functional substituents for use in the present invention as described include, but are not limited to, polyoxyalkylene (polyether), primary and secondary amine, amide, quaternary ammonium, carboxyl, sulfonate, sulfate, carbohydrate, phosphate, and hydroxyl. More preferably, the polar functional substituents of the present invention include, but are not limited to polyoxyalkylene, primary and secondary amine, amide and carboxyl.

A highly preferred class of functionalized silicones which may be used in compositions according to an embodiment of the invention are those containing polyoxyalkylene polar functional substituents.

The polyoxyalkylene content (% polyether) should be from 5 to 42%, preferably from 10 to 40%, and more preferably from 15 to 35%. Preferably, the sum of % silicone and % polyether does not total 100%, other constituents, such as amine and amide making up the balance. The silicone content is defined above and the polyether content (% polyether) is defined as the molecular weight of each polyether pendant or block multiplied by the average number of pendants or blocks and divided by the average molecular weight of the whole polymer. If the pendant or block polyether comprises of both ethylene oxide (EO) and propylene oxide (PO) units, then this % polyether comprises the summation of % EO and % PO. If the pendant or block polyether is comprised of either only EO or only PO units, this % polyether is equivalent to the %EO or %PO, respectively.

The above-defined functionalized silicones with polyoxyalkylene polar functional substituents are capable of depositing evenly and durably on a fibrous substrate. Without wishing to be limited by theory, the above-defined percentages of polyether and silicone impart the polarity characteristics to the functionalized silicone to achieve both sufficient durability and deposition evenness simultaneously.

More preferably still, the functionalized silicones of the present invention are those of the pendant type comprising amino and polyoxyalkylene groups of the average formula (1):

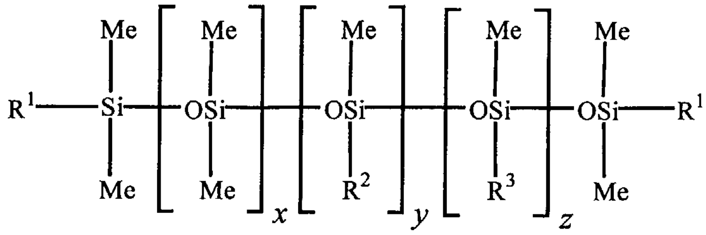

(1)

where Me equals methyl; $R^1$ is methyl or $R^2$ or $R^3$; $R^2$ is $-(CH_2)_a-NH-[(CH_2)_a-NH]_b-H$; and $R^3$ is $-(CH_2)_a-(OC_2H_4)_m-(OC_3H_6)_n-OZ$; wherein x is about 50 to 1500, y is about 1 to 20, z is about 1 to 20; a is about 2 to 5, preferably 2 to 4; b is 0 to 3, preferably 1; m is about 1 to 30; n is about 1 to 30, and Z is H, an alkyl group with 1-4 carbons, or an acetyl group, with the proviso that when y is 0, $R^1$ is an $R^2$ group, and when z is 0, $R^1$ is an $R^3$ group.

The following exemplary structures may be prepared according to the invention:

Organomodified Silicone Example A

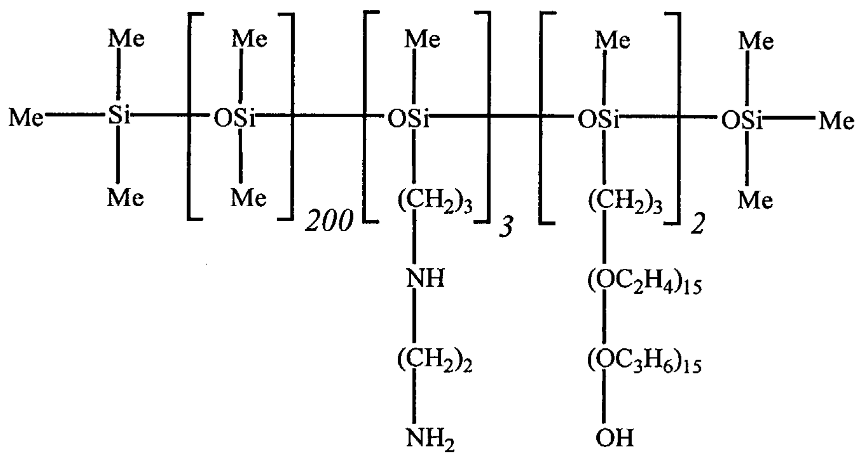

In Example A, molecular weight = 18738; % oxygen = 5.21% (contributed by thirty one oxygen atoms); and

| | |
|---|---|
| % silicone = | 81.42% |
| % polyether = | 16.33% |
| % otherf* = | 2.25% |
| | 100.00% |

*contributed by other side chain and the $-(CH_2)_3-$ and -OH moieties on the polyether side chain.

Organomodified Silicone Example B

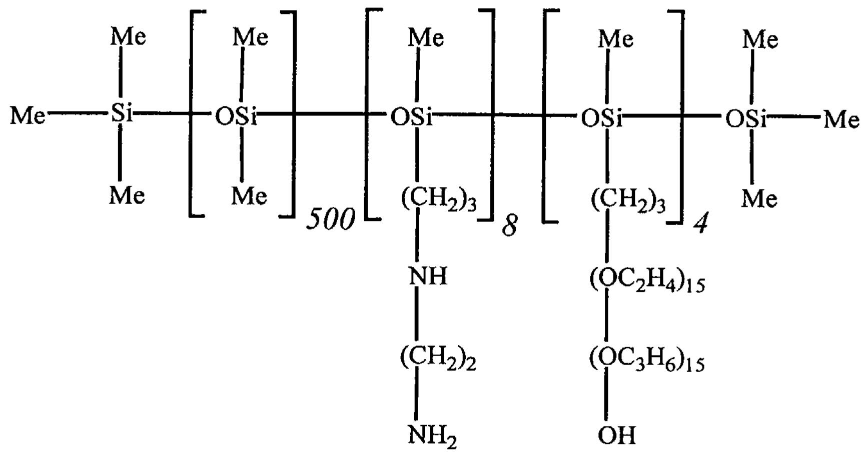

Where Me is methyl.

According to a second aspect of the invention, a fiber treatment composition is provided comprising a functionalized silicone as defined in the first aspect of the invention. Fiber treatment compositions according to the second aspect of the invention are capable of depositing the functionalized silicones according to the first aspect of the invention onto fibrous substrates in a durable and even fashion, which current fiber treatment compositions have been unable to do.

Fiber treatment compositions according to the invention may comprise from 0.1 to 20wt%, preferably from 0.50 to 15wt%, more preferably from 0.50 to 10wt% and more preferably still from 0.50 to 7.5wt% functionalized silicone.

Other advantageous modifications of the first aspect of the invention are discussed hereinbelow and defined in the attached claims.

According to a third aspect of the invention, a hair treatment kit is provided comprising:

(a) an oxidative bleaching composition
(b) a dye composition

and a composition, as defined in the second aspect of the invention, within component (a) and/or within component (b) and/or as a separate component.

For comparison with the functionalized silicones according to the invention, the results of NMR analysis of a number of commercially available silicones are given in the below Table 1 together with the measured hydrophilicity indexes and performance data:

Table 1

| | **HI** | **% Sil** | **% Polyether** | **% Oxygen** | **Deposition Evenness (%)** | **Durability Index** |
|---|---|---|---|---|---|---|
| Wetsoft CTW[1] | 100.0 | 41 | 58 | 18 | 70% | 0.0 |
| Abilsoft AF100[3] | 99.7 | 56 | 43 | 14 | 110 | 0.0 |
| XS69-B5476[2] | 99.5 | 65 | 34 | 11 | 250 | 0.1 |
| DC8211[5] | 73.4 | 95 | 0 | 0 | 5 | 1.2 |
| KF862[6] | 60.2 | 97 | 0 | 0 | 5 | 1.4 |
| KF-861[6] | 55.8 | 96 | 0 | 0 | 6 | 1.5 |
| DC2-8822[5] | 45.7 | 97 | 0 | 0 | 5 | 1.4 |

[1]Wacker Silicones
[2]GE-Bayer Silicones
[3]Goldschmidt
[5]Dow Corning
[6]Shin-Etsu.

Table 1 (continued)

| | HI | % Sil | % Polyether | % Oxygen | Deposition Evenness (%) | Durability Index |
|---|---|---|---|---|---|---|
| Rhodorsil 21637[4] | 37.4 | 98 | 0 | 0 | 3 | 1.4 |
| ADM1100[1] | ** | 99 | 0 | 0 | 3 | 4.2 |

** Not measurable via Hydrophilicity Index measurement

[1]Wacker Silicones

[4]Rhodia

Pendant organomodified silicones comprising amino and polyoxyalkylene groups of the average formula (1), above, can be prepared by methods known to those skilled in the art, via steps including known polymerisation reactions (e.g. equilibration or polycondensation) and known methods of introducing organic substitution to the silicone backbone (e.g. hydrosilation).

The fiber treatment composition according to the present invention may include a cosmetically acceptable vehicle to act as a diluent, dispersant, or carrier for the silicone oil in the composition, so as to facilitate the distribution of the silicone oil when the composition is applied. The vehicle may be an aqueous emulsion, water, liquid or solid emollients, solvents, humectants, propellants, thickeners and powders.

Advantageously, the fiber treatment compositions according to the present invention may be in the form an emulsion with water as a primary component, although aqueous organic solvents may also be included. The emulsion may be a water-in-oil emulsion, an oil-in-water emulsion, a water-in-oil-in-water multiple emulsion, or an oil-in-water-in-oil multiple emulsion, but is preferably an oil-in-water emulsion (a silicone-in-water emulsion). In such a case the functionalized silicone particle size is preferably greater than 500nm, more preferably greater than 1μm and even more preferably greater than 2μm.

The aqueous continuous phase of the emulsion treatment compositions of the present invention may further comprise an emulsifier to facilitate the formation of the emulsion. Emulsifiers for use in the aqueous continuous phase of the present emulsion treatment compositions may include an anionic surfactant, cationic surfactant, amphoteric surfactant, water-soluble polymeric surfactant, water-soluble silicone-containing surfactant, nonionic surfactant having an HLB of greater than about 10, or a surfactant system capable of forming stabilizing liquid crystals around the silicone droplets. The nonionic surfactant preferably has an HLB of at least 12, and more preferably, an HLB value of at least about 15. Surfactants belonging to these classes are listed in McCutcheon's Emulsifiers and Detergents, North American and International Editions, MC Publishing Co., Glen Rock NJ, pages 235-246 (1993).

The emulsifier for the aqueous phase does not gel the aqueous phase. The emulsifier however may be capable of forming a stabilizing layer of lamellar liquid crystals around silicone droplets. This barrier film prevents coalescence between emulsion droplets. In this case, the surfactant system can be a single surfactant or a blend of surfactants. In some cases, a particular surfactant cannot form a liquid crystal structure alone, but can participate in the formation of liquid crystals in the presence of a second surfactant. Such a surfactant system forms a layer of lamellar liquid crystals around the silicone to provide a barrier between the silicone and the aqueous phase. This type of an emulsion is different from the conventional emulsions, which rely upon the orientation of the hydrophobic and hydrophilic components of a surfactant at an silicone-water interface. The formation of a layer of lamellar liquid crystals around the silicone can be detected by the presence of Maltese crosses viewed by optical microscopy through crossed polarizing plates or by freeze fracture electron microscopy.

Exemplary classes of surfactants capable of participating in the formation of a liquid crystal structure around the silicone droplets include, but are not limited to specific cationic surfactants, anionic surfactants, nonionic surfactants, quaternary ammonium surfactants and lipid surfactants.

Preferred surfactants for the formation of liquid crystals in the aqueous continuous phase are of the nonionic type and include $C_{16-22}$ fatty alcohols, and $C_{16-22}$ fatty alcohol ethoxylates with 1 to 30 ethylene oxide groups.

Specific examples include cetearyl alcohol, cetyl alcohol, stearyl alcohol, arachidyl alcohol, oleyl alcohol, ceteareth ethoxylates with between 10 and 30 ethylene oxide groups, ceteth ethoxylates with between 10 to 30 ethylene oxide groups, steareth ethoxylates with between 10 and 30 ethoxylates, and combinations thereof. Preferably, $C_{16-22}$ fatty alcohols are used in combination with $C_{16-22}$ fatty alchol ethoxylates at a ratio of between 10:1 to 0.5:1, more preferably between 6:1 and 1:1, and most preferably between 5:1 and 1.5:1.

The aqueous continuous phase should ideally comprise the emulsifier in an amount sufficient to stabilize the silicone. In one embodiment, the aqueous continuous phase comprises the emulsifier in an amount of from about 0.1% to about 15%, and more preferably from about 0.1% to about 10%, based on the weight of the aqueous continuous phase.

The composition according to the present application finds particular utility in hair coloring compositions especially oxidative hair colorants wherein the hair is subjected to a particularly aggressive environment.

A preferred hair coloring agent for use herein is an oxidative hair coloring agent. The concentration of each oxidative hair coloring agent in the compositions according to the present invention may be from about 0.0001% to about 5% by weight.

Any oxidative hair coloring agent can be used in the compositions herein. Typically, oxidative hair coloring agents comprise at least two components, which are collectively referred to as dye forming intermediates (or precursors). Dye forming intermediates can react in the presence of a suitable oxidant to form a colored molecule.

The dye forming intermediates used in oxidative hair colorants include: aromatic diamines, aminophenols, various heterocycles, phenols, napthols and their various derivatives. These dye forming intermediates can be broadly classified as; primary intermediates and secondary intermediates. Primary intermediates, which are also known as oxidative dye precursors, are chemical compounds which become activated upon oxidation and can then react with each other and/or with couplers to form colored dye complexes. The secondary intermediates, also known as color modifiers or couplers, are generally colorless molecules which can form colors in the presence of activated precursors/primary intermediates, and are used with other intermediates to generate specific color effects or to stabilise the color.

Primary intermediates suitable for use in the compositions and processes herein include: aromatic diamines, polyhydric phenols, amino phenols and derivatives of these aromatic compounds (e.g., N-substituted derivatives of the amines, and ethers of the phenols). Such primary intermediates are generally colorless molecules prior to oxidation.

While not wishing to be bound by any particular theory, it is believed that the process by which color is generated from these primary intermediates and secondary coupler compounds generally includes a stepwise sequence whereby the primary intermediate can become activated (by oxidation), and then enjoins with a coupler to give a dimeric, conjugated colored species, which in turn can enjoin with another 'activated' primary intermediate to produce a trimeric conjugated colored molecule.

In general terms, oxidative dye primary intermediates include those materials which, on oxidation, form oligomers or polymers having extended conjugated systems of electrons in their molecular structure. Because of the new electronic structure, the resultant oligomers and polymers exhibit a shift in their electronic spectra to the visible range and appear colored. For example, oxidative primary intermediates capable of forming colored polymers include materials such as aniline, which has a single functional group and which, on oxidation, forms a series of conjugated imines and quinoid dimers, trimers, etc. ranging in color from green to black. Compounds such as p-phenylenediamine, which has two functional groups, are capable of oxidative polymerization to yield higher molecular weight colored materials having extended conjugated electron systems. Oxidative dyes known in the art can be used in the compositions according to the present invention. A representative list of primary intermediates and secondary couplers suitable for use herein is found in Sagarin, "Cosmetic Science and Technology"," Interscience, Special Ed. Vol. 2 pages 308 to 310.

The primary intermediates can be used alone or in combination with other primary intermediates, and one or more can be used in combination with one or more couplers. The choice of primary intermediates and couplers will be determined by the color, shade and intensity of coloration which is desired. There are nineteen preferred primary intermediates and couplers which can be used herein, singly or in combination, to provide dyes having a variety of shades ranging from ash blonde to black; these are: pyrogallol, resorcinol, p-toluenediamine, p-phenylenediamine, o-phenylenediamine, m-phenylenediamine, o-aminophenol, p-aminophenol, 4-amino-2-nitrophenol, nitro-p-phenylenediamine, N-phenyl-p-phenylenediamine, m-aminophenol, 2-amino-3-hydroxypyridine, 1-napthol, N,N bis (2-hydroxyethyl)p-phenylenediamine, resourcinol, diaminopyrazole, 4-amino-2-hydroxytoluene, 1,5-dihydroxynapthalene, 2-methyl resorcinol and 2,4-diaminoanisole. These can be used in the molecular form or in the form of peroxide-compatible salts.

The hair coloring compositions of the present invention may, in addition to or instead of an oxidative hair coloring agent, include non-oxidative and other dye materials. Optional non-oxidative and other dyes suitable for use in the hair coloring compositions and processes according to the present invention include both semi-permanent, temporary and other dyes. Non-oxidative dyes as defined herein include the so-called 'direct action dyes', metallic dyes, metal chelate dyes, fiber reactive dyes and other synthetic and natural dyes. Various types of non-oxidative dyes are detailed in: 'Chemical and Physical Behaviour of Human Hair' 3rd Ed. by Clarence Robbins (pp250-259); 'The Chemistry and Manufacture of Cosmetics'. Volume IV. 2nd Ed. Maison G. De Navarre at chapter 45 by G.S. Kass (pp841-920); 'cosmetics: Science and Technology' 2nd Ed., Vol. II Balsam Sagarin, Chapter 23 by F.E. Wall (pp 279-343); 'The Science of Hair Care' edited by C. Zviak, Chapter 7 (pp 235-261) and .'Hair Dyes', J.C. Johnson, Noyes Data Corp., Park Ridge, U.S.A. (1973), (pp 3-91 and 113-139).

The hair coloring compositions herein preferably comprise at least one oxidising agent, which may be an inorganic or organic oxidising agent. The oxidising agent is preferably present in the coloring composition at a level of from about 0.01% to about 10%, preferably from about 0.01% to about 6%, more preferably from about 1% to about 4% by weight of the composition.

A preferred oxidising agent for use herein is an inorganic peroxygen oxidising agent. The inorganic peroxygen oxidising agent should be safe and effective for use in the present compositions. Preferably, the inorganic peroxygen

oxidising agents suitable for use herein will be soluble in the compositions according to the present invention when in liquid form or in the form intended to be used. Preferably, inorganic peroxygen oxidising agents suitable for use herein will be water-soluble. Water soluble oxidising agents as defined herein means agents which have a solubility to the extent of about 10g in 1000ml of deionised water at 25°C ("Chemistry" C. E. Mortimer. 5th Edn. p277).

The inorganic peroxygen oxidising agents useful herein are generally inorganic peroxygen materials capable of yielding peroxide in an aqueous solution. Inorganic peroxygen oxidising agents are well known in the art and include hydrogen peroxide, inorganic alkali metal peroxides such as sodium periodate, sodium perbromate and sodium peroxide, and inorganic perhydrate salt oxidising compounds, such as the alkali metal salts of perborates, percarbonates, perphosphates, persilicates, persulphates and the like. These inorganic perhydrate salts may be incorporated as monohydrates, tetrahydrates etc. Mixtures of two or more of such inorganic peroxygen oxidising agents can be used if desired. While alkali metal bromates and iodates are suitable for use herein the bromates are preferred. Highly preferred for use in the compositions according to the present invention is hydrogen peroxide.

The compositions herein may instead or in addition to the inorganic peroxygen oxidising agent(s), comprise one or more preformed organic peroxyacid oxidising agents.

Suitable organic peroxyacid oxidising agents for use in the coloring compositions according to the present invention have the general formula:

$$R - C(O)OOH$$

wherein R is selected from saturated or unsaturated, substituted or unsubstituted, straight or branched chain, alkyl, aryl or alkaryl groups with from 1 to 14 carbon atoms.

The organic peroxyacid oxidising agents should be safe and effective for use in the compositions herein. Preferably, the preformed organic peroxyacid oxidising agents suitable for use herein will be soluble in the compositions used according to the present invention when in liquid form and in the form intended to be used. Preferably, organic peroxyacid oxidising agents suitable for use herein will be water-soluble. Water-soluble preformed organic peroxyacid oxidising agents as defined herein means agents which have a solubility to the extent of about 10g in 1000ml of deionised water at 25°C ("Chemistry" C. E. Mortimer. 5th Edn. p277).

The compositions herein may optionally contain a transition metal containing catalyst for the inorganic peroxygen oxidising agents and the optional preformed peroxy acid oxidising agent(s). Suitable catalysts for use herein are disclosed in WO98/27945.

The compositions herein may contain as an optional component a heavy metal ion sequestrant. By heavy metal ion sequestrant it is meant herein components which act to sequester (chelate or scavenge) heavy metal ions. These components may also have calcium and magnesium chelation capacity, but preferably they show selectivity to binding heavy metal ions such as iron, manganese and copper. Such sequestering agents are valuable in hair coloring compositions as herein described for the delivery of controlled oxidising action as well as for the provision of good storage stability of the hair coloring products.

Heavy metal ion sequestrants may be present at a level of from about 0.005% to about 20%, preferably from about 0.01% to about 10%, more preferably from about 0.05% to about 2% by weight of the compositions.

Suitable sequestering agents are disclosed in WO98/27945.

For use, the treatment compositions according to an embodiment of the invention may be provided at a pH from about 3 to 11, preferably from 4 to 10.5.

The present compositions do not only find application in the treatment of fibers, such as hair, but may also be applied to other substrates, such as human skin, nails and various animal body parts, such as horns, hooves and feathers.

## TEST METHODS

### Viscosity of functionalized silicone fluids - measurement protocol

An AR 500 rotational rheometer (TA Instruments Ltd., Leatherhead, Surrey KT22 7UQ, UK) is used to determine the viscosity of the functionalized silicone fluids used herein. The determination is performed at 30°C, with the 4cm 2° steel cone measuring system set with a 49µm (micron) gap and is performed via the programmed application of a shear stress of 0.5 to 590 Pa over a 2 minute time period. These data are used to create a shear rate vs. shear stress curve for the material. This flow curve can then be modelled in order to provide a material's viscosity. These results were fitted with the following well-accepted Newtonian model:

Viscosity, $\mu = \sigma/\gamma$

(where σ is shear stress; γ is shear rate)

Method for assessing silicone particle size within a product

A microscope (Nikon Eclipse E800) is utilised to determine the silicone particle size in the final product. Typically, pictures are taken (JVC color video camera KY-F50) of the final product at a magnification ranging from 100x to 400x. Using the captured image a scale is superimposed (Image software - Lucia G Version 4.51 (build 028), Laboratory Imaging) previously calibrated using a 100 μm Graticule (Graticules Ltd, Tonbridge Wells, Kent, England) and compared to the average silicone particle within the sample to provide an estimation of particle size.

**Functional Silicone Deposition and Deposition Evenness Determination Method**

Hair substrate preparation method

Two different hair substrates of differing polarities are required upon which to measure silicone deposition, and thereby also calculate a value for deposition evenness across the two substrates. Hair is supplied by Hugo Royer International Limited (10 Lakeside Business Park, Sandhurst, Berkshire, GU47 9DN, England) and is a blended, Eastern European, mid-brown human hair. Prior to use, the hair is assessed and qualified for low cuticular damage (<20%) and misalignment (<5%), based on at least 200 hair strands per batch. Any damage on a hair strand counts as one point damaged, and then the total is calculated as a percentage. This hair is made into 4" (10cm), 2g round tied switches (where the length and weight of hair corresponds to the hair below the tie). To obtain hair substrates with two distinct polarities this hair is then pre-treated according to one of two distinct protocols.

Virgin hair preparation

Hair switches are washed in a sink fitted with a shower attachment set with a flow rate of 6 ± 1 L $min^{-1}$ and a temperature of 37 ± 2 °C using the following protocol: switches are initially wetted under the shower attachment for 30 s. The hair is then removed from the water flow and 0.2 g of shampoo (Pantene Classic Care Shampoo) is applied down each switch, and then lathered for 30s by hand before rinsing for 60s under the shower. The hair is again removed from the shower, and has a further 0.2 g of shampoo applied, and lathered for 30 s before finally rinsing under the shower for 60s. Hair switches are then left to dry in a controlled temperature cabinet set at 30°C. This washing protocol comprising two shampoo applications and one drying step is defined as a single wash cycle. After this wash cycle is completed, the hair is then defined hereinafter as "virgin" hair and is used hereinafter as a hydrophobic hair substrate.

Chemically damaged hair preparation

Hair switches are chemically damaged using the following two component bleaching formulations:

| **Peroxide base** | |
|---|---|
| Ingredients | Wt/Wt% |
| 1. Emulsion base: | |
| Deionized water | 29.78 |
| Cetyl alcohol (1) | 2.24 |
| Stearyl alcohol (2) | 2.24 |
| Ceteareth-25 (3) | 1.50 |
| Phenoxyethanol (4) | 0.11 |
| Sodium benzoate (5) | 0.09 |
| Tetrasodium EDTA (87%) (6) | 0.04 |
| | |
| 2. Chelant premix | |
| Deionized water | 35.72 |

(continued)

| Peroxide base | |
|---|---|
| 2. Chelant premix | |
| Pentasodium pentetate (40%) (7) | 0.24 |
| Hydroxyethane diphosphonic acid | 0.16 |
| (60%) (8) | |
| Phosphoric acid (75%) (9) | 0.08 |
| Sodium stannate (95%) (10) | 0.04 |
| | |
| 3. Peroxide mix | |
| Hydrogen peroxide (35%) (11) | 17.15 |
| Deionized water | 10.61 |

| **Carrier base for dye base** | |
|---|---|
| Ingredients | Wt/Wt% |
| 1. Acetic acid pre-mix | |
| Deionized water | 46.49 |
| Acetic acid (50%) (12) | 3.91 |
| | |
| 2. Emulsion base | |
| Deionized water | 29.78 |
| Cetyl alcohol (1) | 2.24 |
| Stearyl alcohol (2) | 2.24 |
| Ceteareth-25 (3) | 1.50 |
| Phenoxyethanol (4) | 0.11 |
| Sodium benzoate (5) | 0.09 |
| Tetrasodium EDTA (87%) (6) | 0.04 |
| | |
| Ammonium hydroxide (13) | 13.60 |

(1): available as Surfac cetyl alcohol from Surfachem, Leeds, UK
(2): available as Surfac stearyl alcohol from Surfachem, Leeds, UK
(3): available as Volpo CS25 from Croda, North Humberside. UK
(4): available as Phenoxyethanol from Nipa-Hardwicke, Wilmington, Delaware
(5): available as Sodium benzoate EP/USP from Haltermann, Cumbria, UK
(6): available as Edeta B powder from BASF, Cheadle, Cheshire, UK
(7): available as Trilon C liquid from BASF, Cheadle, Cheshire, UK
(8): available as Dequest 2010 from Solutia, Newport, South wales
(9): available as Phosphoric acid 750F from Albright & Wilson, West Midlands, UK
(10): available as Sodium stannate, Aldrich
(11): available as Hydrogen peroxide 35% 171/4 from Ellis & Everard, Walsall, UK
(12): available as 50% acetic acid from Hays, Greenwich, London, UK
(13): available as Ammonium Solution BP grade from Brotherton Speciality Products, West Yorkshire, UK

These products are made using the following protocols:

Peroxide base:

The first stage is to make the emulsion base; this is prepared by adding to a vessel deionized water and commencing agitation, and then heating to 82°C. Then tetrasodium EDTA and sodium benzoate are added and dissolved, followed by addition of ceteareth25, cetyl alcohol and stearyl alcohol. During the addition process the temperature is maintained above 80°C, finally phenoxyethanol is added, the mixture is then homogenized for 30 min. The emulsion structure is obtained by cooling whilst still high shear mixing the product down below 50°C. The emulsion base is then left to thicken for 60 min.

The chelants are added to the deionised water with mixing to form the chelant premix. This is then added with stirring to the pre-made emulsion base. Adding the peroxide mix water followed by hydrogen peroxide to the emulsion base/chelant premix and stirring until homogeneous makes the completed peroxide base.

Carrier base for dyes

The carrier base for dyes is prepared by adding water to a vessel and commencing agitation, followed by the addition of acetic acid, then by the emulsion base (see emulsion base preparation described hereinbefore for the peroxide base). When fully mixed, ammonium hydroxide is added to the mixture and the stirring continued until the product is homogenous.

To use this bleaching system, equal weights of the two components, the peroxide base and carrier base for dyes are mixed together thoroughly. To each dry untreated hair switch, 4g of this bleaching system is then applied, and thoroughly worked into the hair, using the fingers, to ensure even, complete, coverage. The hair switch is then wrapped in cling film and incubated in an oven at 30°C for 30 minutes, after which the product is rinsed for 2 minutes (in a sink fitted with a shower attachment set with a flow rate of 6 ± 1 L $min^{-1}$ and a temperature of 37 ± 2 °C ) with finger agitation. Finally the switches are dried using a hot air drier (Babyliss Lightweight Professional model 1015 (1400 W)) for 3 min. The bleached hair switches are then washed in a sink fitted with a shower attachment set with a flow rate of 6 ± 1 L $min^{-1}$ and a temperature of 37 ± 2 °C. Switches are initially wetted under the shower attachment for 30s. The hair is then removed from the water flow and 0.2 g of shampoo (Pantene Clarifying Shampoo) is applied down each switch, and then lathered for 30 s by hand before rinsing for 60 s under the shower. The hair is again removed from the shower, and has a further 0.2g of shampoo applied, and lathered for 30s before finally rinsing under the shower for 60s. Hair switches are then dried using a hot air drier (Babyliss Lightweight Professional model 1015 (1400 W)) for 3min. This washing protocol comprising two shampoo applications and one drying step is defined as a single wash cycle. This washing method is then repeated again through another complete wash cycle. The dry hair switches are then bleached again according to the method outlined above and subsequently washed again through two complete wash cycles. This hair is hereinafter defined as "damaged" hair and is hereafter used a hydrophilic hair substrate.

Hair treatment

The functionalized silicone under investigation for deposition and deposition evenness is prepared for assessment using the following method. 28.8 g of the peroxide base, described hereinbefore for use in the preparation of the damaged hair substrate, is weighed into a 100 ml glass beaker; 1.2 g of silicone is then added to the vessel along with a 25 mm magnetic flea and placed on a magnetic stirrer (IKA RCTbasic) and left for 30 min at a stirring rate of 1000 rpm. This product is then removed from the magnetic stirrer, and 30 g of the carrier base for dyes, described hereinbefore in the preparation of the damaged hair substrate, is then added and thoroughly mixed until homogenous using hand agitation via a plastic spatula. 16 g of the bleaching system containing the silicone under investigation is then applied simultaneously to two virgin and two damaged hair switches (equal to 4 g for each individual switch), held together in the same clamp, and thoroughly worked into the hair, using the fingers, to ensure even, complete, coverage. The hair is then wrapped in cling film and incubated in an oven at 30 °C for 30 minutes after which it is then rinsed for 2 minutes (in a sink fitted with a shower attachment set with a flow rate of 6 ± 1 L $min^{-1}$ and a temperature of 37 ± 2 °C) with finger agitation. The switches are dried using a hot air drier (Babyliss Lightweight Professional model 1015 (1400 W)) for 3 min.

Deposition measurement

A wavelength dispersive X-Ray Fluoresence spectrometer (Phillips Electronics, PW2404 Sequential "4000W" X-Ray Spectrometer System) is utilised to determine the silicone deposition level on hair. The spectrometer is fitted

with a Rhodium tube and includes an InSb crystal to facilitate high sensitivity silicone detection.

Characteristic x-ray photons are produced from the ejection of an inner shell electron of an silicone atom followed by a transition of an electron from a higher energy state to the empty inner shell. X-ray fluorescence of silicone in polydimethylsiloxane (PDMS) is directly proportional to the amount of PDMS deposited on the hair. Any deviations from this relationship that may occur for more functionalized silicones cancels out on calculation of the Deposition Evenness Value (below). A critical component to facilitate the use of XRF technology is the ability to present the sample to the spectrometer in a consistent manner. The hair switch is arranged in a custom-made sample holder, which presents a continuous, flat, aligned hair surface across the exposed sample area (16 mm diameter). The sample is analysed under a helium atmosphere using a Tube voltage of 32 kV and current of 125 mA, with an irradiation/acquisition time of 60 s.

The drift in the analytical signal is regularly monitored and evaluated. The preferred approach employed is to use a known standard that does not need to be prepared each time the drift is assessed. An Ausmon sample is an appropriate monitor sample for many applications, including silicon determinations. A drift correction with the Ausmon sample for silicon is performed at the beginning of each day samples are analyzed. The calculated drift is below 3% between sets of analyses.

Calculation of the amount of silicon on hair in units of ppm from can be made with equation 1.

$$x_2=(I-b_1)/m_1 \qquad (1)$$

Where $m_1$ and $b_1$ are calculated from a calibration curve constructed from measurements of the XRF signal as a function of the amount of silicone deposited on hair subsequently assayed using atomic absorption on the extracted silicone.

To translate the XRF silicone deposition data obtained as hereinbefore described into a measure of deposition evenness on hair substrates prepared with different levels of chemical damage, it is necessary to generate a deposition evenness value. To generate the deposition evenness value the following equation is employed:

$$\text{Deposition Evenness Value (\%)} = \frac{\text{Dep(1)}}{\text{Dep(2)}} \text{x100}$$

Where Dep(1) equals the XRF deposition value obtained on "damaged" hair (preparation of described hereinbefore), Dep(2) equals the XRF deposition value obtained on "virgin" hair (preparation of described hereinbefore).

## Silicone Durability Index Method

### Hair substrate preparation

Durability is only assessed on a polar, chemically damaged substrate. Hair is supplied by Hugo Royer International Limited (10 Lakeside Business Park, Sandhurst, Berkshire, GU47 9DN, England) and is a blended, Eastern European, mid-brown human hair. Prior to use the hair is assessed and qualified for low cuticular damage (<20%) and misalignment (<5%), based on at least 200 hair strands per batch. Any damage on a hair strand counts as one point damaged, and then the total is calculated as a percentage. This hair is made into 10cm (4"), 2 g round tied switches (where the length and weight of hair corresponds to the hair below the tie). To obtain a damaged, polar hair substrate the following protocol is used.

In the following procedure, the peroxide base, chelant premix and carrier base for dyes employed are as above for the "Functional Silicone Deposition and Deposition Evenness Determination Method". In addition, the methods for preparation of the "Peroxide base" and "Carrier base for dyes" are also identical.

### Hair treatment

Silicones are deposited on to the hair via a solvent matrix. Propan-2-ol (obtained from Aldrich Chemicals, product # 15,479-2) is used as the solvent in the delivery of functional silicone fluids herein. The silicone fluid is solubilized in 2-propanol at a concentration of 0.20 % using a magnetic stirrer. Hair tresses are laid flat on cling film and the resulting 2-propanol/silicone solution applied using a syringe at a dosage of 1g silicone solution/ 1g of hair (half to each side). The solution is then massaged into the hair using fingers for 30 s. The treated switches are allowed to dry naturally in the ambient atmosphere. When the switches are dry they are split into two groups both comprising equal numbers of damaged hair switches. The first are used to measure the initial deposition after the 2-propanol deposition.

The second set is washed to assess the silicone durability. The hair switches are washed in a sink fitted with a shower attachment set with a flow rate of 6 ± 1 L $min^{-1}$ and a temperature of 37 ± 2 °C. Switches are initially wetted under the shower attachment for 30 s. The hair is removed from the water flow and 0.2 g of shampoo ("Pantene Classic Clean Shampoo") is applied along each switch, and then lathered for 30 s by hand before rinsing for 60 s under the shower. The switch then has a further 0.2 g of shampoo application, and is lathered for 30 s before finally rinsing under the shower for 60 s. Hair switches are then dried using a hot air drier (Babyliss Lightweight Professional model 1015 (1400 W)_ for 3 min. This protocol comprising two shampoo applications and one drying step is defined as one complete wash cycle. This washing protocol is then repeated again through another eleven complete cycles (to make twelve wash cycles in total). These switches are then measured for silicone deposition to assess the durability performance.

Deposition measurement

Measurement of deposition uses XRF values, as described above for the "Functional Silicone Deposition and Deposition Evenness Determination Method".

To translate the XRF silicone deposition data obtained as hereinbefore described into a measure of silicone durability, it is necessary to generate a silicone durability index value. To generate the silicone durability index value the following equation is employed:

$$\text{Silicone durability index value} = \frac{Dep(12cycle)}{Dep(initial)}$$

Where Dep(initial) equals the XRF deposition value obtained on hair after silicone deposition with no washing cycles, Dep(12cycles) equals the XRF deposition value obtained on hair after silicone deposition and subsequent 12 wash cycles.

EXAMPLES

The following examples further describe and demonstrate the preferred embodiments within the scope of the present invention. The examples are given solely for the purpose of illustration, and are not to be construed as limitations of the present invention since many variations thereof are possible without departing from its scope.

Examples 1-3 - Colorant compositions

| **Peroxide base** | **#1** | **#2** | **#3** |
|---|---|---|---|
| Ingredients | Wt% | Wt% | Wt% |
| Emulsion base: | | | |
| Deionized water | 29.17 | 29.17 | 29.17 |
| Cetyl alcohol (1) | 2.20 | 2.20 | 2.20 |
| Stearyl alcohol (2) | 2.20 | 2.20 | 2.20 |
| Ceteareth-25 (3) | 1.47 | 1.47 | 1.47 |
| Phenoxyethanol (4) | 0.11 | 0.11 | 0.11 |
| Sodium benzoate (5) | 0.09 | 0.09 | 0.09 |
| Tetrasodium EDTA (87%) (6) | 0.04 | 0.04 | 0.04 |
| | | | |
| | | | |
| Deionized water | 35.00 | 35.00 | 35.00 |
| Pentasodium pentetate (40%) (7) | 0.24 | 0.24 | 0.24 |
| Hydroxyethane diphosphonic acid (60%) (8) | 0.16 | 0.16 | 0.16 |

(continued)

| **Peroxide base** | **#1** | **#2** | **#3** |
|---|---|---|---|
| Ingredients | Wt% | Wt% | Wt% |
| Emulsion base: | | | |
| Phosphoric acid (75%) (9) | 0.08 | 0.08 | 0.08 |
| Sodium stannate (95%) (10) | 0.04 | 0.04 | 0.04 |
| | | | |
| Hydrogen peroxide (35%) (11) | 16.80 | 16.80 | 16.80 |
| Deionized water | 10.40 | 10.40 | 9.40 |
| | | | |
| Silicone of formula A | 0 | 2.00 | 0 |
| Silicone of formula B | 2.00 | 0 | 3.00 |

| **Carrier base for dye base** | **#1** | **#2** | **#3** |
|---|---|---|---|
| Ingredients | Wt% | Wt% | Wt% |
| | | | |
| Deionized water | 46.49 | 46.49 | 46.49 |
| Acetic acid (50%) (12) | 3.91 | 3.91 | 3.91 |
| Emulsion base (see ingredients above) | 36.00 | 36.00 | 36.00 |
| Ammonium hydroxide (13) | 13.60 | 13.60 | 13.60 |

(1): available as Surfac cetyl alcohol from Surfachem, Leeds, UK
(2): available as Surfac stearyl alcohol from Surfachem, Leeds, UK
(3): available as Volpo CS25 from Croda, North Humberside. UK
(4): available as Phenoxyethanol from Nipa-Hardwicke, Wilmington, Delaware
(5): available as Sodium benzoate EP/USP from Haltermann, Cumbria, UK
(6): available as Edeta B powder from BASF, Cheadle, Cheshire, UK
(7): available as Trilon C liquid from BASF, Cheadle, Cheshire, UK
(8): available as Dequest 2010 from Solutia, Newport, South wales
(9): available as Phosphoric acid 750F from Albright & Wilson, West Midlands, UK
(10): available as Sodium stannate, Aldrich
(11): available as Hydrogen peroxide 35% 171/4 from Ellis & Everard, Walsall, UK
(12): available as 50% acetic acid from Hays, Greenwich, London, UK
(13): available as Ammonium Solution BP grade from Brotherton Speciality Products, West Yorkshire, UK

Production of the example colorant applications

Peroxide base:

The emulsion base is prepared by adding to a vessel the deionized water and commencing agitation with heating to 82°C. Then the preservatives (tetrasodium EDTA, sodium benzoate) are added and dissolved. This is followed by addition of ceteareth25, cetyl alcohol and stearyl alcohol while keeping the temperature above 80°C. Then phenoxytol is added. The mixture is then fully blended hot through a recirculation line and homogenized. The emulsion structure is obtained by cooling the product down below 50°C and shearing while cooling. The product is left to thicken for 60min.

The chelant premix is prepared by adding the chelants to water and mixing them together in a vessel. Then this solution is added to the emulsion base. The completed peroxide base is made by adding water to the previous

mixture followed by the hydrogen peroxide while stirring.

To this peroxide base the silicone can then be added and stirred.

Carrier system for dye base :

The carrier base is prepared by adding water to a vessel and commencing agitation, followed by the addition of acetic acid. Then emulsion base (see emulsion base preparation described above) is added. When fully homogenized, ammonium hydroxide is added to the mixture.

Examples 4-5 - After colorant conditioners

| | **#4** | **#5** |
|---|---|---|
| Ingredients | Wt% | Wt% |
| | | |
| Deionized water | 61.95 - qs | 60.95 -qs |
| | | |
| Emulsion base: | | |
| Deionized water | 29.76 | 29.76 |
| Cetyl alcohol (1) | 2.25 | 2.25 |
| Stearyl alcohol (2) | 2.25 | 2.25 |
| Ceteareth-25 (3) | 1.50 | 1.50 |
| Phenoxyethanol (4) | 0.11 | 0.11 |
| Sodium benzoate (5) | 0.09 | 0.09 |
| Tetrasodium EDTA (87%) (6) | 0.04 | 0.04 |
| | | |
| Citric acid anhydrous fine (14) | pH trim | pH trim |
| | | |
| Silicone of formula A | 2.00 | 0 |
| Silicone of formula B | 0 | 3.00 |
| (14): available as citric acid anhydrous fine from Aldrich | | |

Composition preparation

The conditioner composition is prepared by adding to a vessel the deionized water and the emulsion base (see emulsion base preparation described above) while stirring. When homogenized citric acid is added to the mixture until the pH of the emusltion is between 5 and 6.

Then the single fluids can then be added to the emulsion and stirred.

### Claims

1. Functionalized silicone of the pendant or graft type having a viscosity in the range 50 to 150,000 mPa.s and incorporating one or more polar substituents selected from electron withdrawing, electron neutral, or electron donating groups with Hammett sigma para values between -1.0 and +1.5, wherein the one or more polar substituents comprise oxygen, such that the oxygen content of the summation of the one or more polar substituents is from 1% to 10% of the weight of the functionalized silicone and the silicone content is from 67 to 95% of the weight of the functionalized silicone.

**2.** Functionalized silicone according to claim 1 having a viscosity in the range 400 - 100,000 mPa.s, preferably from 4000 to 25,000 mPa.s.

**3.** Functionalized silicone according to claim 1 or 2, having the following formula:

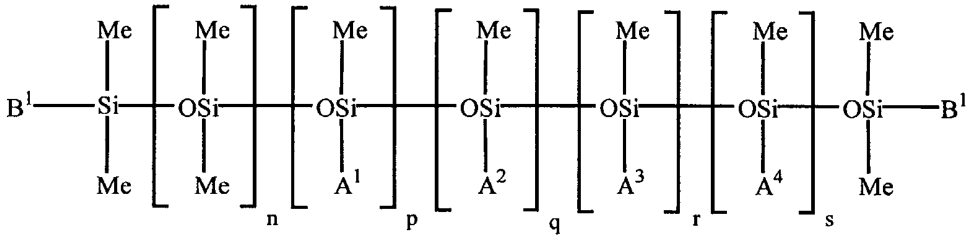

or a block copolymer type according to the following formula:

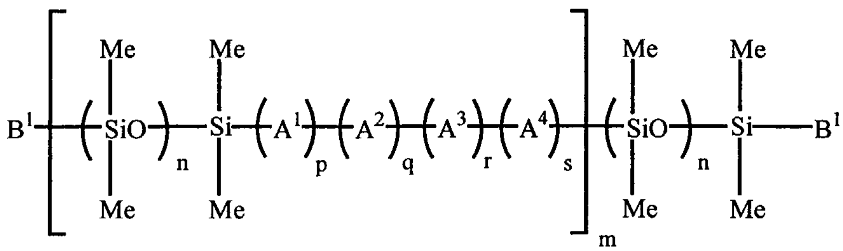

where Me is a methyl group, m is greater than or equal to 1, n is about 50 to 2000, p is about 0 to 50, q is about 0 to 50, r is about 0 to 50, s is about 0 to 50, wherein p + q + r + s is greater than or equal to 1, $B^1$ is H, OH, an alkyl or an alkoxy group and organic groups $A^1$, $A^2$, $A^3$ and $A^4$ are straight, branched or mono- or polycyclic aliphatic, mono or polyunsaturated alkyl, aryl, heteroalkyl, heteroaliphatic or heteroolefinic moieties comprising 3 to 150 carbon atoms together with 0-50 heteroatoms incorporating the one or more polar substituents.

**4.** Functionalized silicone according to claim 3, wherein the polar substituents comprise groups $\alpha^1$, $\alpha^2$, $\alpha^3$, and $\alpha^4$ as defined below; S-linked groups including $S\alpha^1$, SCN, $SO_2\alpha^1$, $SO_3\alpha^1$, $SS\alpha1^1$, $SO\alpha^1$, $SO_2N\alpha^1\alpha^2$, $SN\alpha^1\alpha^2$, $S(N\alpha^1)\ \alpha^2$, $S(O)(N\alpha^1)\ \alpha^2$, $S\alpha^1(N\alpha^2)$, $SON\alpha^1\alpha^2$; O-linked groups including $O\alpha^1$, $OO\alpha^1$, OCN, $ON\alpha^1\alpha^2$; N-linked groups including $N\alpha^1\alpha^2$, $N\alpha^1\alpha^2\alpha^3+$, NC, $N\alpha^1O\alpha^2$, $N\alpha^1S\alpha^2$, NCO, NCS, $NO_2$, $N{=}N\alpha^1$, $N{=}NO\alpha^1$, $N\alpha^1CN$, $N{=}C{=}N\alpha^1$, $N\alpha^1N\alpha^2\alpha^3$, $N\alpha^1N\alpha^2N\alpha^3\alpha^4$, $N\alpha^1N{=}N\alpha^2$; COX, $CON_3$, $CON\alpha^1\alpha^2$, $CON\alpha^1CO\alpha^2$, $C({=}N\alpha^1)N\alpha^1\alpha^2$, CHO, CHS, CN, NC, and X, where:

$\alpha^1$, $\alpha^2$, $\alpha^3$, and $\alpha^4$ are straight, branched or mono- or polycyclic aliphatic, mono or polyunsaturated alkyl, aryl, heteroalkyl, heteroaliphatic or heteroolefinic moiety comprising 3 to 150 carbon atoms together with 0-50 heteroatoms, especially 0, N, S, P, and

X is F, Cl, Br, or I, where

H is hydrogen, O is oxygen, N is nitrogen, C is carbon, S is sulfur, Cl is chlorine, Br is bromine, I is iodine, F is fluorine.

**5.** Functionalized silicone according to any one of the preceding claims, incorporating silicone branching groups comprising $MeSiO_{3/2}$ or $SiO_{4/2}$ groups.

**6.** Functionalized silicone according to any one of the preceding claims comprising polyoxyalkylene polar substituents.

**7.** Functionalized silicone according to claim 6, wherein the polyoxyalkylene content is between 5 and 42% and the silicone content is from 67 and 95%.

**8.** Functionalized silicone according to claim 6 or 7 having the following formula:

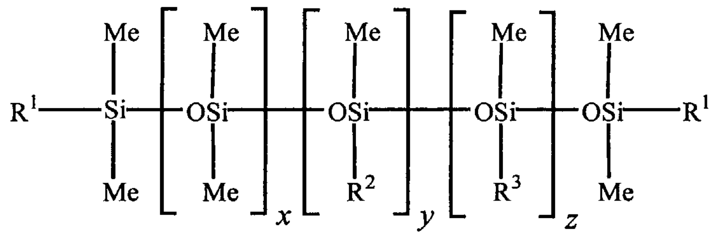

where Me equals methyl; $R^1$ is methyl or $R^2$ or $R^3$; $R^2$ is $-(CH_2)_a-NH-[(CH_2)_a-NH]_b-H$; and $R^3$ is $-(CH_2)_a-(OC_2H_4)_m-(OC_3H_6)_n-OZ$; wherein x is about 50 to 1500, y is about 1 to 20, z is about 1 to 20; a is about 2 to 5, preferably 2 to 4; b is 0 to 3, preferably 1; m is about 1 to 30; n is about 1 to 30, and Z is H, an alkyl group with 1-4 carbons, or an acetyl group, with the proviso that when y is 0, $R^1$ is an $R^2$ group, and when z is 0, $R^1$ is an $R^3$ group.

**9.** Functionalized silicone having the following formula:

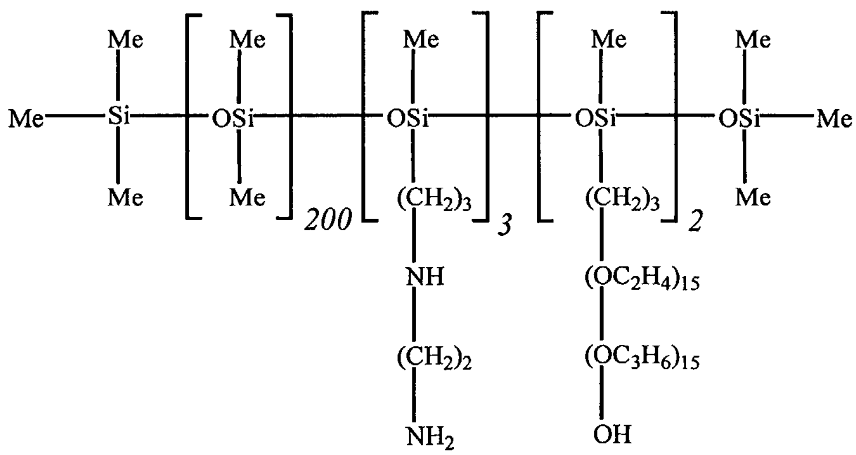

**10.** Functionalized silicone having the formula:

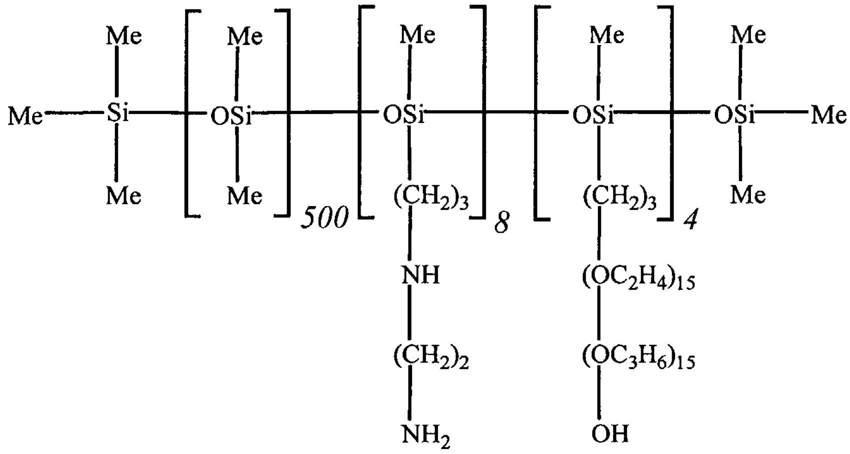

**11.** Fiber treatment composition comprising a functionalized silicone as defined in any one of the preceding claims.

**12.** Fiber treatment composition according to claim 11, wherein the functionalized silicone deposits evenly and durably on the fibers being treated.

**13.** Fiber treatment composition according to any one of claims 11 or 12, wherein the functionalized silicone is present in an amount ranging from 0.1 to 20%wt, preferably from 0.50 to 10%wt.

**14.** Fiber treatment composition according to any one of claims 11 to 13 which is in the form of an oil-in-water emulsion.

**15.** Fiber treatment composition according to claim 14, wherein the silicone droplets dispersed within the aqueous continuous phase have a particle size above 500nm, preferably above 1µm.

**16.** Fiber treatment composition according to claim 14 or 15 additionally comprising 0.1 to 15% based on the weight of the aqueous continuous phase of emulsifier.

**17.** Fiber treatment composition according to claim 16, wherein the emulsifier comprises one or more of an anionic surfactant, cationic surfactant, amphoteric surfactant, water-soluble polymeric surfactant, water soluble silicone-containing surfactant and a non-ionic surfactant.

**18.** Fiber treatment composition according to claim 17, wherein the surfactant comprises $C_{16}$ - $C_{22}$ fatty alcohols and/or fatty alcohol ethoxylates with 1 to 30 ethylene oxide groups, preferably 10 to 30 ethylene oxide groups.

**19.** Fiber treatment composition according to claim 18, wherein the sufactant comprises a mixture of $C_{16-22}$ fatty alcohols and $C_{16-22}$ fatty alchol ethoxylates in a ratio of between 10:1 to 0.5:1, more preferably between 6:1 and 1:1.

**20.** Fiber treatment composition according to any one of claims 11 to 19, wherein the fiber is hair.

**21.** Hair treatment composition according to claim 20 additionally comprising a hair bleaching composition or a hair dyeing composition.

**22.** Hair treatment kit comprising:

(a) an oxidative bleaching composition
(b) a dye composition; and

a hair treatment composition according to claim 19 comprised within component (a) and/or within component (b) and/or provided as a separate component.

European Patent Office

# EUROPEAN SEARCH REPORT

Application Number
EP 03 25 2203

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| D,X | EP 0 275 707 A (DOW CORNING) 27 July 1988 (1988-07-27)<br>* page 4, line 50 - line 53 *<br>* page 5, line 20 - line 25 *<br>* page 5, line 36 - page 6, line 34 *<br>* example 1B *<br>--- | 1-4, 11-14, 16-18, 20-22 | C08G77/14<br>C08G77/26<br>C08G77/38<br>C08G77/46<br>A61K7/13<br>A61K7/135 |
| X | US 5 612 409 A (CHROBACZEK HARALD ET AL) 18 March 1997 (1997-03-18)<br>* claim 1 *<br>* column 12, line 19 - line 33 *<br>* column 14, line 8 - line 35; example 1 *<br>* column 15, line 12 - line 28; example 4 *<br>--- | 1-4,6-8, 11-14, 16-18 | |
| X | US 6 313 256 B1 (O'LENICK JR ANTHONY J) 6 November 2001 (2001-11-06)<br>* claim 1 *<br>* column 8, line 10 - column 11, line 27 *<br>* example 27 *<br>--- | 1-4,6,7, 11,12,20 | **TECHNICAL FIELDS SEARCHED (Int.Cl.7)**<br>C08G<br>A61K |
| X | US 4 246 423 A (MARTIN EUGENE R) 20 January 1981 (1981-01-20)<br>* claims 11-14 *<br>* column 11, line 25 - column 12, line 14; examples 5,6 *<br>* column 13, line 9 - line 48; examples 9,10 *<br>----- | 1,2,5-7, 11-14 | |

The present search report has been drawn up for all claims

| Place of search | Date of completion of the search | Examiner |
|---|---|---|
| MUNICH | 29 July 2003 | Öhm, M |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

## ANNEX TO THE EUROPEAN SEARCH REPORT ON EUROPEAN PATENT APPLICATION NO. EP 03 25 2203

This annex lists the patent family members relating to the patent documents cited in the above-mentioned European search report.
The members are as contained in the European Patent Office EDP file on
The European Patent Office is in no way liable for these particulars which are merely given for the purpose of information.

29-07-2003

| Patent document cited in search report | | Publication date | Patent family member(s) | | | Publication date |
|---|---|---|---|---|---|---|
| EP 0275707 | A | 27-07-1988 | US | 4749732 | A | 07-06-1988 |
| | | | AU | 605533 | B2 | 17-01-1991 |
| | | | AU | 8318187 | A | 07-07-1988 |
| | | | CA | 1302289 | C | 02-06-1992 |
| | | | DE | 3779417 | D1 | 02-07-1992 |
| | | | EP | 0275707 | A2 | 27-07-1988 |
| | | | JP | 1754542 | C | 23-04-1993 |
| | | | JP | 4040325 | B | 02-07-1992 |
| | | | JP | 63218612 | A | 12-09-1988 |
| US 5612409 | A | 18-03-1997 | DE | 4222483 | A1 | 13-01-1994 |
| | | | AT | 161861 | T | 15-01-1998 |
| | | | BR | 9302789 | A | 16-02-1994 |
| | | | DE | 59307912 | D1 | 12-02-1998 |
| | | | DK | 578144 | T3 | 07-09-1998 |
| | | | EP | 0578144 | A2 | 12-01-1994 |
| | | | ES | 2111666 | T3 | 16-03-1998 |
| | | | JP | 7010999 | A | 13-01-1995 |
| US 6313256 | B1 | 06-11-2001 | NONE | | | |
| US 4246423 | A | 20-01-1981 | AT | 4723 | T | 15-10-1983 |
| | | | AU | 528669 | B2 | 05-05-1983 |
| | | | AU | 6044280 | A | 30-04-1981 |
| | | | CA | 1159073 | A1 | 20-12-1983 |
| | | | DE | 3064963 | D1 | 27-10-1983 |
| | | | EP | 0028357 | A1 | 13-05-1981 |
| | | | JP | 2045647 | B | 11-10-1990 |
| | | | JP | 56062824 | A | 29-05-1981 |
| | | | US | 4293611 | A | 06-10-1981 |



For more details about this annex : see Official Journal of the European Patent Office, No. 12/82